# EUROPEAN PATENT SPECIFICATION

(11) **EP 3 196 292 B1**
(45) Date of publication and mention of the grant of the patent: **22.04.2020**
(21) Application number: 15841887.1
(22) Date of filing: 14.08.2015
(51) Int. Cl.: C12M 3/00, C12N 5/07, C12N 5/10, C12M 1/32

(54) **CELL CULTURE APPARATUS AND METHOD**
ZELLKULTURVORRICHTUNG UND -VERFAHREN
DISPOSITIF ET PROCÉDÉ DE CULTURE CELLULAIRE

(30) Priority: 18.09.2014 JP 2014189622
(43) Date of publication of application: 26.07.2017
(73) Proprietor: Fujifilm Corporation, Minato-ku Tokyo 106-8620 (JP)
(72) Inventor: WAKUI Takashi, Kanagawa 258-8538 (JP)
(74) Representative: Klunker IP Patentanwälte PartG mbB
(86) International application number: PCT/JP2015/072957
(87) International publication number: WO 2016/042956

(56) References cited:
- EP-A1- 2 586 872
- WO-A1-03/044158
- WO-A1-2011/043077
- WO-A1-2011/089908
- WO-A1-2011/161962
- WO-A1-2012/098931
- JP-A- 2002 296 205
- JP-A- 2002 345 456
- JP-A- 2002 360 242
- JP-A- 2012 147 693
- JP-A- 2014 075 999
- US-A1- 2008 317 324
- US-A1- 2013 027 539
- DATABASE WPI Week 200332 Thomson Scientific, London, GB; AN 2003-336370 XP002773116, -& JP 2002 360242 A (JAPAN TISH ENG KK) 17 December 2002 (2002-12-17)
- KOIKE, H. ET AL.: 'Establishment of automated culture system for murine induced pluripotent stem cells' BMC BIOTECHNOLOGY vol. 12, no. 81, 2012, pages 1 - 8, XP021121085
- LEONARDO, T.R. ET AL.: 'Culturing Human Pluripotent Stem Cells on a Feeder Layer' HUMAN STEM CELL MANUAL 2012, pages 3 - 14, XP008184337

## Description

### BACKGROUND OF THE INVENTION

### 1. Field of the Invention

The present invention relates to a cell culture device and a cell culture method that respectively culture a plurality of cell colonies in a plurality of cell culture portions.

### 2. Description of the Related Art

Recently, clinical studies for forming living tissues for transplantation by culturing embryonic stem (ES) cells and induced pluripotent stem (iPS) cells, performing differentiation induction on cells such as skins or cartilages, and using the differentiation induced cells are conducted.

In order to form cell sheets such as skins for transplantation and cartilages for transplantation, first, a small amount of cells are gathered from living bodies, the gathered cells are induced to undifferentiated cells such as iPS cells, and the obtained undifferentiated cells are cultured. The cell sheets are formed by differentiating and inducing the obtained cells to skins or cartilages, making sheets with the differentiated and induced cells or rearranging the differentiated and induced cells in a three-dimensional structure.

Meanwhile, when the cells are cultured, images of the cells are picked up and proliferation properties or qualities of the cells are evaluated based on the images. For example, in WO2007/052716A, it is suggested that the cultivation states of the cells are determined based on the images obtained by picking up images of the cells, and the predetermined cultivation operations are performed based on the determination results. In WO2009/031283A, it is suggested that morphological information indicating cultivation states of the cells, information on a rate of changes, or the like are obtained by interpreting images obtained by picking up images of the cells.

US 2013/027539 A1 discloses a cell observing apparatus and a cell incubation method. In the cell incubation method, between cell observation steps, target cells are transferred from one incubation container into another incubation container.

JP 2002-360242 A discloses a method for judging the suitability for transplantation of cultured tissue. In the method, the total mass of a plurality of cell culture units is measured, the measurement is compared with a transplantability reference, and based on the result, the suitability for transplantation of the cultured tissue is judged.

EP 2586872 A1 discloses a method and device for identifying multipotent stem cell colonies, and for automatic culturing of multipotent stem cells.

WO 03/044158 A1 discloses methods and devices for the integrated discovery of cell culture environments.

### SUMMARY OF THE INVENTION

Here, when living tissues for transplantation as described above are formed, maturity of respective cells (proliferation properties or qualities) varies during the cultivation process of cells used regardless of autologous cells and heterologous cells. In a case where these cells with variation are made to sheets or three-dimensionally rearranged, qualities as living tissues are deteriorated in some cases. There is a problem in that it is difficult to stably supply living tissues for transplantation having constant qualities as products.

WO2007/052716A and WO2009/031283A do not suggest methods for solving the problem described above.

In view of the above, an object of the invention is to provide a cell culture device and cell culture method that enable living tissues for transplantation having constant qualities to be stably supplied as products.

A cell culture device according to the invention comprises: a plurality of cell culture portions operable to respectively culture a plurality of cell culture units; a maturity evaluation portion operable to respectively evaluate maturity of the respective cell culture units present in the respective cell culture portions during operation of the cell culture device; a movement target cell determining portion operable to determine a cell culture unit that does not satisfy a predetermined condition as a target to be moved to another cell culture portion in a case where the cell culture unit of which maturity does not satisfy the predetermined condition among a plurality of cell culture units cultured in any one cell culture portion of the plurality of cell culture portions exists; and a cell moving portion operable to move the cell culture unit determined as the target to be moved to the other cell culture portion, wherein the movement target cell determining portion is operable to determine the cell culture unit as the target to be moved to another cell culture portion based on the sizes of the evaluation values, and wherein the movement target cell determining portion is operable to cause the maturity of the respective cell culture units to be even by determining the respective cell culture units cultured in the respective cell culture portions among a plurality of cell culture portions as the target to be moved.

It is preferable that the cell culture device is operable to set culture conditions of at least two cell culture portions to be different from each other.

It is preferable that the cell culture device is operable to set start times of culturing at least two cell culture portions to be different from each other.

It is preferable that the cell culture device is operable to set culture conditions that influence on culture speeds of at least two cell culture portions to be different from each other.

Preferably, in the cell culture device, the conditions that influence on culture speeds include at least one of culture medium concentrations, types of culture media, exchange frequencies of culture media, types of scaffolding, scaffolding applying concentrations to culture media, temperature, humidity, types of light sources, illuminance of light sources, shaking condition, oxygen concentration, or carbon dioxide concentrations.

Preferably, in the cell culture device, the maturity evaluation portion is operable to evaluate maturity of the respective cell culture units based on at least one of the number of cells, cell density, shapes of cells, sizes of cells, movement speeds of cells, proliferation speeds of cells, the number of cell colonies, sizes of cell colonies, information on shapes of cell colonies, which are obtained during operation of the cell culture device based on images of the respective cell culture units, or information on components discharged from cells obtained by interpreting culture medium components of the respective cell culture units.

Preferably, in the cell culture device, the maturity evaluation portion is operable to evaluate maturity based on images obtained by capturing cell culture units at two or more types of magnification.

Preferably, in the cell culture device, the movement target cell determining portion is operable to determine whether maturity of the respective cell culture units satisfies the predetermined condition by using a standard deviation or a variance of maturity of all of the cell culture units cultured in any one cell culture portion during operation of the cell culture device.

It is preferable that the cell culture device further comprises:
an evaluation standard storage unit operable to store relationships between the culture periods of the cell culture units and evaluation standards of maturity corresponding to the culture periods, wherein the movement target cell determining portion is operable to obtain the evaluation standard as the predetermined condition based on a present culture period and the relationships.

It is preferable that the cell culture device is operable to set start times of culturing at least two cell culture portions to be different from each other, and in a case where a cell culture unit of which maturity is lower than the predetermined condition exists during operation of the cell culture device, the movement target cell determining portion is operable to determine the cell culture unit that does not satisfy the condition as a target to be moved to a cell culture portion of which the start timing of the culture is later than the any one cell culture portion.

It is preferable that the cell culture device is operable to set culture conditions that influence on culture speeds of at least two cell culture portions to be different from each other, in a case where a cell culture unit of which maturity is lower than the predetermined condition exists during operation of the cell culture device, the movement target cell determining portion is operable to determine that a cell culture unit that does not satisfy the condition is a target to be moved to a cell culture portion having a culture condition of which a culture speed is faster than that of the any one cell culture portion.

Preferably, in the cell culture device, in a case where a cell culture unit that does not satisfy both of the predetermined condition and a condition different from the predetermined condition exists during operation of the cell culture device, the cell moving portion is operable to remove the cell culture unit that does not satisfy the both conditions from the cell culture portion.

It is preferable that the cell culture device further comprises:
a notification portion operable to notify information of the cell culture unit determined as a target to be moved.

Preferably, in the cell culture device, the cell culture unit present during operation of the cell culture device is a cell colony unit, a well unit in a culture vessel, or a culture vessel unit.

It is preferable that the cell culture device further comprises:
a high speed culture portion operable to culture the cell culture unit of which maturity does not satisfy the predetermined condition until the maturity reaches the condition and that is a cell culture portion adapted to have a culture speed which is a higher speed than those of the plurality of cell culture portions.

A cell culture method according to the invention, respectively culturing a plurality of cell culture units in a plurality of cell culture portions; respectively evaluating maturity of the respective cell culture units in the respective cell culture portions; automatically determining a cell culture unit that does not satisfy a predetermined condition as a cell culture unit to be moved to another cell culture portion in a case where the cell culture unit of which maturity does not satisfy the predetermined condition among a plurality of cell culture units cultured in any one cell culture portion of the plurality of cell culture portions exists; and moving the cell culture unit determined as the target to be moved to the other cell culture portion, wherein in a step of determining the cell culture unit, the cell culture unit is determined as the target to be moved to another cell culture portion based on the sizes of the evaluation values, and wherein in a step of determining the cell culture unit, the maturity of the respective cell culture units is caused to be even by determining the respective cell culture units cultures in the respective cell culture portions among a plurality of cell culture portions as the target to be moved.

It is preferable, that the cell culture method further comprises:
culturing the cell culture unit of which maturity does not satisfy the predetermined condition until the maturity reaches the condition in a high speed culture portion which is a cell culture portion of which a culture speed is a higher speed than those of the plurality of cell culture portions.

According to the cell culture device and the cell culture method according to the invention, a plurality of cell culture units are respectively cultured in a plurality of cell culture portions, and maturity of the respective cell culture units in the respective cell culture portions is respectively evaluated. In a case where cell culture units of which maturity does not satisfy the predetermined condition among a plurality of cell culture units cultured in the same cell culture portions exist, the cell culture units that do not satisfy the condition are determined as cell culture units to be moved to other cell culture portions.

Accordingly, in a case where cell culture units of which maturity is low or maturity is high among the plurality of cell culture units cultured in the same cell culture portions exist, the cell culture units are moved to the other cell culture portions in which maturity of the cell culture units based on the determination is different, such that maturity of a plurality of cell culture units in the respective cell culture portions can be caused to be even. Accordingly, if the living tissues for transplantation are formed by using a plurality of cell culture units cultured by the respective cell culture portions, living tissues for transplantation having constant qualities can be stably supplied as products.

### BRIEF DESCRIPTION OF THE DRAWINGS

Fig. 1 is a block diagram schematically illustrating a configuration of a cell culture device according to a first embodiment of the invention.
Fig. 2 is a diagram illustrating an example of a culture vessel having a plurality of wells.
Fig. 3 is a diagram illustrating an example of a function indicating a relationship between a culture period and a threshold value corresponding to the culture period.
Fig. 4 is a flow chart for describing an operation of the cell culture device according to the first embodiment of the invention.
Fig. 5 is a diagram schematically illustrating a flow from the start of seeding of cell colonies in first to third cell culture portions to making the cell colonies after culture into sheets and packaging the cell colonies to be shipped.
Fig. 6 is a diagram illustrating movement of wells (cell culture units) between cell culture portions in the cell culture device according to the first embodiment.
Fig. 7 is a diagram for describing movement of wells (cell culture units) between cell culture portions in the cell culture device according to a second embodiment.
Fig. 8 is a block diagram schematically illustrating another embodiment of the cell culture device according to the invention.
Fig. 9 is a diagram schematically illustrating an embodiment in a case where suspension culture is performed.

### DESCRIPTION OF THE PREFERRED EMBODIMENTS

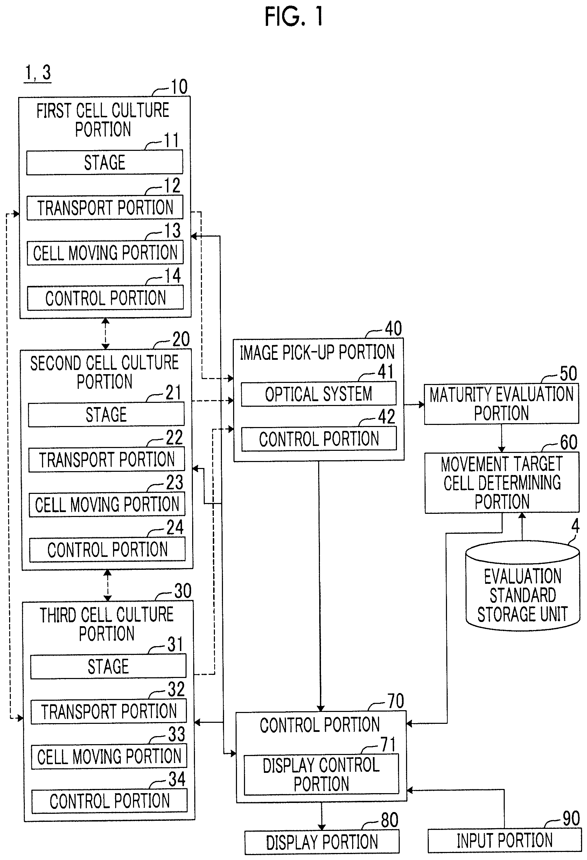

Hereinafter, a cell culture device and a cell culture method according to a first embodiment of the invention will be described with respect to the drawings. Fig. 1 is a block diagram schematically illustrating a cell culture device 1 according to the embodiment.

As illustrated in Fig. 1, the cell culture device 1 according to the embodiment includes first to third cell culture portions 10, 20, and 30, an image pick-up portion 40, a maturity evaluation portion 50, a movement target cell determining portion 60, a control portion 70, a display portion 80, and an input portion 90.

The first to third cell culture portions 10, 20, and 30 culture cells. As cells to be cultured, any arbitrary cells can be used regardless of derived animal species or types of organs or tissues, as long as the cells are proliferate under a predetermined culture condition. Specific examples thereof include primary cells collected from livers, pancreas, kidneys, nerves, skins, or fat derived from humans or animals other than humans (for example, monkeys, pigs, dogs, rats, or mice), mouse iPS cells initialized by introducing four factors of Oct-4, Sox2, Klf4, and c-Myc, mouse iPS cells initialized with three factors of Oct-4, Sox2, and Klf4, human iPS cells initialized by introducing four genes of OCT-4, SOX2, NANOG, and LIN28 to fetal lung-derived fibroblasts or neonatal foreskin-derived fibroblasts, human iPS cells initialized by introducing four genes of OCT-4, SOX2, NANOG, and LIN28 to fetal lung-derived fibroblasts or neonatal foreskin-derived fibroblasts, undifferentiated stem cells, embryo-derived ES cells, vascular endothelium cells, created established cell lines, or cells obtained by genetically manipulating these cells. Differentiation induced cells of nerves, skins, cardiac muscles, liver, or the like, or cancer cells or the like can be preferably used.

In the respective cell culture portions 10, 20, and 30, one or a plurality of culture vessels in which cells to be cultured are seeded in a culture medium are stored. Since basic configurations of the first to third cell culture portions 10, 20, and 30 according to the embodiment are the same, configurations of the first cell culture portion 10 are mainly described. The first cell culture portion 10 includes a stage 11, a transport portion 12, a cell moving portion 13, and a control portion 14.

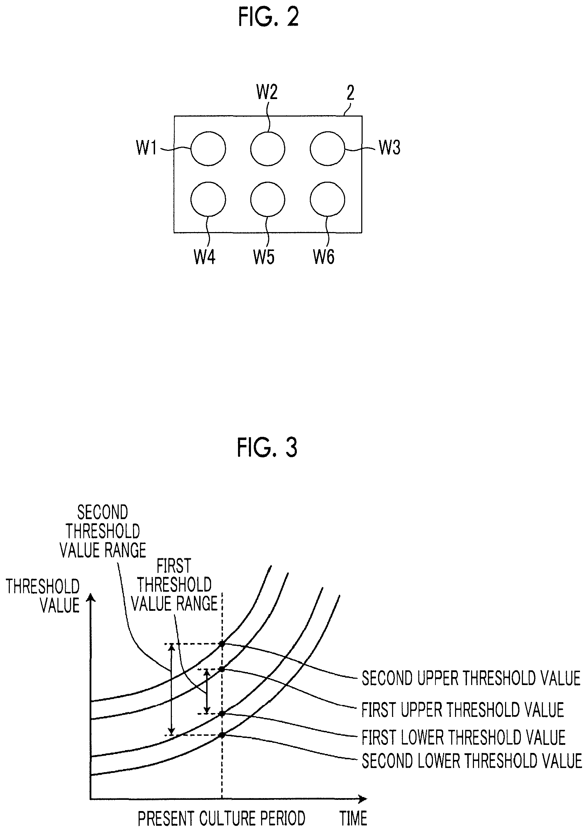

In the stage 11, a culture vessel in which cells to be cultured are seeded is installed. Fig. 2 illustrates an example of a culture vessel 2 used in the embodiment. As illustrated in Fig. 2, the culture vessel 2 used in the embodiment is a well plate including a plurality of wells W1 to W6. A culture medium and one or a plurality of colonies of cells to be cultured are stored in each of the wells W1 to W6, and thus the cell colonies are cultured in the respective wells W1 to W6. In the embodiment, the respective wells W1 to W6 correspond to cell culture units according to the invention. The stage 11 may include a mechanism that shakes the culture vessel 2.

When images of the cell colonies in respective wells in the culture vessel 2 are picked up by the image pick-up portion 40, the transport portion 12 transports the culture vessel 2 from the first cell culture portion 10 to an image pick-up position in the image pick-up portion 40. Specific configurations of the transport portion 12 may be, for example, a configuration of including a rotation stage and a driving mechanism that rotates the rotation stage thereof, and transporting the culture vessel 2 that is moved from the stage 11 to the rotation stage to the image pick-up portion 40 by rotating a rotation stage by a driving mechanism. The transport portion 12 may have a configuration of including a transport belt and a driving mechanism thereof and transporting the culture vessel 2 to the image pick-up portion 40 by the transport belt. The transport portion 12 may have a configuration of including a transport robot and transporting the culture vessel 2 to the image pick-up portion 40 by the transport robot.

In a case where wells of which maturity does not satisfy predetermined conditions based on evaluation results on maturity of the cell colonies in the respective wells W1 to W2 in the maturity evaluation portion 50 exist, the cell moving portion 13 moves the wells to other cell culture portions. In a case where maturity of the wells is extremely deviated from predetermined conditions or in a case where a cell culture portion does not exist at a movement target, the cell moving portion 13 moves cell colonies to a vessel for disposal (not illustrated) in the well and disposes the cell colonies. A case where the cell culture portion does not exist at the movement target is described below.

As the specific configuration of the cell moving portion 13, for example, the cell moving portion 13 may include a robot arm and select and move a well with the robot arm to other cell capture portions. As the methods of moving the respective wells, for example, in a case of a configuration in which the respective wells W1 to W6 of the culture vessels 2 can be detached, the wells may be grabbed and moved by the robot arm, or cell colonies in the respective wells W1 to W2 are sucked by a sucking mechanism together with a culture medium and the sucked cell colonies may be moved to other cell culture portions by the robot arm. A well to be moved is determined by the movement target cell determining portion 60, but the determination method is described below.

The control portion 14 controls the entire first cell culture portion 10 and controls operations from the start of the culture to the end of the culture in the first cell culture portion 10. Specifically, the control portion 14 controls culture conditions such as temperature and humidity in the first cell culture portion 10, a type of a light source used, illuminance of the light source, an oxygen concentration, a carbon dioxide concentration, and shaking conditions of the stage 11 or the like. With respect to the configuration of adjusting the culture conditions thereof, well-known configurations can be used. The control portion 14 also controls operations of the transport portion 12 or the cell moving portion 13.

Basic configurations of the second cell culture portion 20 and the third cell culture portion 30 are the same as those of the first cell culture portion 10. That is, configurations of the stages 11, 21, and 31 are the same with each other, configurations of the transport portions 12, 22, and 32 are the same with each other, configurations of the cell moving portions 13, 23, and 33 are the same with each other, and configurations of the control portions 14, 24, and 34 are the same with each other.

The image pick-up portion 40 picks up images of the respective wells W1 to W6 of the culture vessel 2. The image pick-up portion 40 includes an optical system 41 that picks up images of the respective wells W1 to W6 and outputs image signals and a control portion 42 that controls the optical system 41.

The optical system 41 includes a microscope such as a phase contrast microscope, a differential interference microscope, or a bright field microscope. These microscopes include imaging elements such as complementary metal-oxide semiconductor (CMOS) sensors or charge-coupled device (CCD) sensors, and image signals obtained by picking up images of the inside of the respective wells W1 to W6 are output from these imaging elements.

The control portion 42 controls the entire image pick-up portion 40. Particularly, according to the embodiment, the control portion 42 controls optical magnification of the optical system 41. Control of optical magnification of the optical system 41 is described below.

The images of the respective wells W1 to W6 picked up by the image pick-up portion 40 are input to the maturity evaluation portion 50, and the maturity evaluation portion 50 evaluates maturity of the cell colonies included in the images based on the images thereof. Specifically, the maturity evaluation portion 50 according to the embodiment specifies cell colonies included in the images obtained by picking up images of the respective wells W1 to W6, and sizes of the cell colonies are obtained as evaluation values of the maturity. As the size of the cell colonies, for example, areas, perimeters, or maximum diameters of the cell colonies can be obtained. As a method of specifying a cell colony, a cell colony can be specified by binarizing the picked up image by using a threshold value of luminance, but other well-known methods may be used. As a method of measuring sizes of the cell colonies, well-known methods can be used.

For example, in a case where a plurality of cell colonies in one well are included, the maturity evaluation portion 50 obtains an average value, a maximum value, a minimum value, a median value, or the like of sizes of the plurality of cell colonies as an evaluation value. However, in a case where the respective wells are used as cell culture units as in the embodiment, it is desirable to seed one cell colony in each of the wells. In a case where the plurality of cell colonies are seeded, the plurality of cell colonies are preferably seeded in an arrangement in which the cell colonies do not be bound to each other, in view of maturity evaluation accuracy.

According to the embodiment, sizes of the cell colonies are obtained as an evaluation value of maturity of cell colonies, but the evaluation values are not limited to sizes of the cell colonies, other feature values may be obtained as evaluation values of maturity. For example, the number of cells in a cell colony, the number of cells for each unit area smaller than a cell colony (cell density), a shape of a cell, a size of a cell, a movement speed of a cell, the number of proliferation of a cell per unit time (proliferation speed), the number of cell colonies in a well, or the information of shapes of cell colonies may be obtained as the evaluation value.

In a case where the number of cells in the cell colony or cell density is obtained as an evaluation value, for example, the number of cells may be counted by paying attention to nuclei or nucleoli included in the cells. At this point, for example, the number of cells may be counted by limiting the cells to cells each having one nucleus, cells each having a plurality of nuclei, or cells having nucleoli.

In a case where the sizes of the cells is obtained as the evaluation value, areas, circumference lengths, an average value, a maximum value, a minimum value, or a median value of maximum diameters, of cells included in a well may be obtained as the evaluation value. With respect to a movement speed of the cells, an average value, a maximum value, a minimum value, a median value, or the like of the movement speeds of the cells included in the well may be obtained as an evaluation value. As the method of measuring the movement speed of the cells, for example, with respect to respective cells included in two images obtained by picking up the same well in a times series, the same cells are associated with each other by using pattern matching, and linear movement distances between the associated cells are divided by an image pick-up interval, so as to obtain the movement speed.

With respect to the proliferation speed of the cells, the numbers of cells included in the two images obtained by picking up images of the same well of which images are picked up in a time series are respectively measured, difference of the numbers of cells included in the two images is divided by an image pick-up interval, so as to obtain the proliferation speed of the cells.

With respect to shapes of the cells, circularity of the respective cells included in the images of the wells is calculated, and average values, maximum values, minimum values, median values, or the like of the circularity may be obtained as evaluation values. In addition to the circularity, shape patterns of the cells for each type of the cells to be cultured are stored in advance, similarity between the respective cells included in the images and the shape patterns is calculated, average values, maximum values, minimum values, median values, or the like of the similarity may be obtained as evaluation values. The type information on cells to be cultured may be set and input, for example, by a user using the input portion 90.

For example, textures such as luminance, evenness, or roughness of the images of the cell colonies may be obtained as evaluation values of maturity. For example, in a case where the cells to be cultured are stem cells, if the maturity of the cells proceeds, concentration degrees of the stem cells increase, further the stem cells are laminated, and the luminance of the images gradually increases. Accordingly, as the luminance is higher, the maturity progresses.

In a case where the maturity progresses and the stem cells grow as described above and become a laminated state, the evenness of the images increases and a smooth image having small irregularity can be obtained. Therefore, as the evenness of the image is higher or the image is smoother, maturity progresses. As a method of obtaining feature amounts of the evenness or the smoothness of the image, well-known methods can be used.

As the evaluation values of the maturity, feature values of the shapes of the cell colonies may be obtained. As the maturity of the cells proceeds, the shapes of the cell colonies gradually become a round shape, and then differentiation of peripheral parts proceeds, and complexity of edges increases. Accordingly, the feature values of the changes of the shapes of the cell colonies like this can be obtained as evaluation values of the maturity.

According to the embodiment, the maturity evaluation portion 50 obtains information such as sizes of the cell colonies described above by interpreting images of the respective wells W1 to W6 but image interpretation does not necessarily performed by the maturity evaluation portion 50. For example, the maturity evaluation portion 50 may obtain results of the image interpretation and may calculate evaluation values of the maturity based on the results.

The information that is used for calculating evaluation values of maturity is not limited to the results of the image interpretation as described above. For example, the maturity evaluation portion 50 may obtain information of components discharged from cells obtained by interpretation of the culture medium components of the respective wells W1 to W6 and calculate evaluation values of maturity based on the information. The components discharged from the cells can be obtained by interpreting components of the old culture medium that is exchanged when culture mediums are exchanged. Specifically, the information of the components discharged from cells can be obtained by measuring electric resistance of the old culture medium components or interpreting reaction of reagents. As the components discharged from the cells are more, it is possible to evaluate that maturity proceeds.

As the evaluation values of the maturity, feature values of the thickness of the cell colonies may be obtained. As the maturity of the cells proceeds, the cell colonies gradually become thick. Accordingly, the feature values of the thickness of the cell colonies like this can be obtained as the evaluation values of the maturity. The thickness of the stem cell colonies can be measured by using, for example, an interferometer such as optical coherence tomography (OCT) (not illustrated).

The maturity evaluation portion 50 according to the embodiment obtains evaluation values in a cell colony unit for obtaining evaluation values from low magnification images described below and obtains evaluation values of the respective cell units for obtaining evaluation values from high magnification images. Specifically, sizes of cell colonies, the number of cell colonies, feature values of shapes of cell colonies (circularity, eccentricity, or the like), average brightness of images of cell colonies, evenness or roughness of images of cell colonies, or average edge strength of images of cell colonies or the like is obtained as evaluation values, for obtaining evaluation values from the low magnification images.

Meanwhile, the maturity evaluation portion 50 obtains the number of cells, cell density, sizes of cells, movement speeds of cells, proliferation speeds of cells, or feature values of shapes of cells in a cell colony as evaluation values, in order to obtain evaluation values from the high magnification images.

The movement target cell determining portion 60 receives an input of evaluation values of maturity of respective wells obtained in the maturity evaluation portion 50, compares the input evaluation values and predetermined conditions with each other, and determines whether the evaluation values satisfy predetermined conditions. Specifically, the movement target cell determining portion 60 according to the embodiment determines whether evaluation values of the maturity of the respective wells are in the range regulated by predetermined threshold values.

With respect to the threshold values described above, functions indicating relationships between culture periods and threshold values (corresponding to evaluation standards according to the invention) according to the culture periods as illustrated in Fig. 3 are stored in the movement target cell determining portion 60 in advance, and the movement target cell determining portion 60 refers to the function based on the present culture period, so as to obtain threshold values. The function may be stored in an evaluation standard storage unit 4 or the like provided as a database different from the cell culture device 1 and be read out from the evaluation standard storage unit 4.

The function may be stored for each type of the cells, such that functions corresponding to types of cells to be cultured are used. For example, a user may set and input the information on types of the cells to be cultured, by using the input portion 90. The present culture period may be measured by providing, for example, a timer or the like, or a user may set and input the present culture period by using the input portion 90.

As a method of obtaining evaluation standards such as threshold values, in addition to a method of using the functions as described above, average values, standard deviation, or variances of evaluation values in all cell culture units cultured in the same cell culture portion, that is, average values, standard deviation, or variances of evaluation of all wells cultured in the same cell culture portion in the case of the embodiment, are calculated as evaluation standards, whether the evaluation values of the maturity of the respective wells are in the range of the calculated standard deviations or variances may be determined. The comparison between the evaluation values of the maturity and threshold values is described below.

For example, in a case where there are wells of which evaluation values thereof are not included in a range regulated by threshold values among the wells W1 to W6 in the culture vessel 2 cultured in the first cell culture portion 10, the movement target cell determining portion 60 determines the wells as a target to be moved to the other second or third cell culture portion 20 or 30. The position information of the wells determined as a target to be moved in the movement target cell determining portion 60 is output to the control portion 70. The method of determining a well to be moved is described below.

The control portion 70 controls the entire cell culture device 1. Particularly, the control portion 70 according to the embodiment obtains position information on a well determined as a target to be moved in the movement target cell determining portion 60, outputs control signals to the control portion 14, 24, or 34 of the first, second, or third cell culture portion 10, 20, or 30 based on the position information, and performs control so as to move the well to be moved to another cell culture portion.

The control portion 70 includes a display control portion 71. The display control portion 71 causes the display portion 80 to display images picked up in the image pick-up portion 40 and causes the display portion 80 to display evaluation results in the maturity evaluation portion 50 and position information of the well determined as the target to be moved in the movement target cell determining portion 60. The display portion 80 is formed by a display device such as a liquid crystal display.

As a method of displaying position information of a well determined as a target to be moved, for example, the display portion 80 is caused to display a schematic diagram of the entire culture vessel 2 and the wells W1 to W6 as illustrated in Fig. 2 and only a range of a well determined as a target to be moved is caused to have a different display mode such as a display by changing a color different from ranges of the other wells. A user can be informed of a well to be moved by performing a display as above.

According to the embodiment, as described above, the well to be moved is automatically moved. However, the user may manually move the well by performing a display as described above.

According to the embodiment, the display control portion 71 corresponds to a notification portion according to the invention. The method of notifying a well to be moved to a user is not limited to the display above, for example, a lamp that indicates a position of the well to be moved may be turned on or a position of the well may be informed by voice. A display portion or a lamp is provided on the stage 11 on which the culture vessel 2 is installed, and a user is informed of a position of the well to be moved by the display portion or the lamp. In this case, for example, a configuration in which the culture vessel 2 is installed on a schematic display of the culture vessel as illustrated in Fig. 2, and positions of the wells in the culture vessel 2 and the position of the well to be moved are associated with each other is desirable.

The input portion 90 includes a mouse, a keyboard, or the like and receives a setting input by a user. The input portion 90 according to the embodiment receives input of setting and change of the evaluation standard described above and receives an input of a change of a culture condition in a case where the culture condition of first to third cell culture portion 10, 20, or 30 is changed. The display portion 80 may also function as the input portion 90 such as the display portion 80 is made to a touch panel and setting is input by pressing a screen of the touch panel.

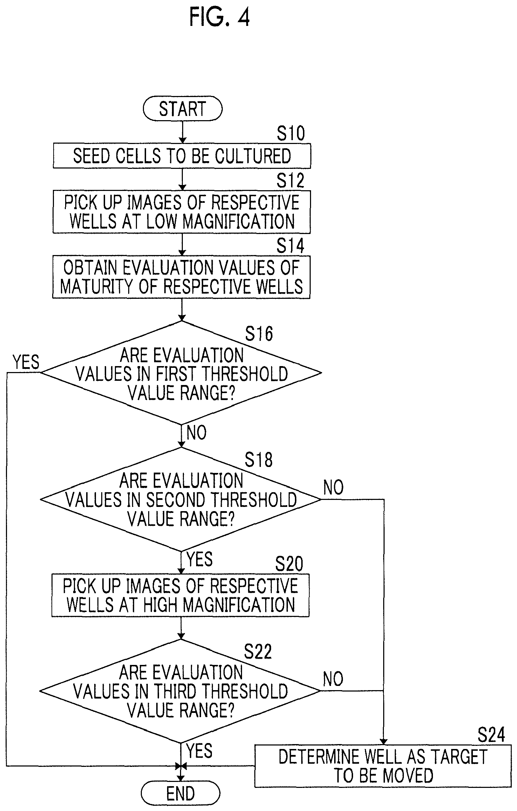

Subsequently, the operation of the cell culture device 1 according to the embodiment is described with reference to a flow chart illustrated in Fig. 4. Here, a flow from the start of seeding of cell colonies, to a step of making a sheet with the cell colonies after culture and package shipment is described. The evaluation of maturity of the cell colonies in the middle of the culture and movement of wells based on evaluation results thereof are described.

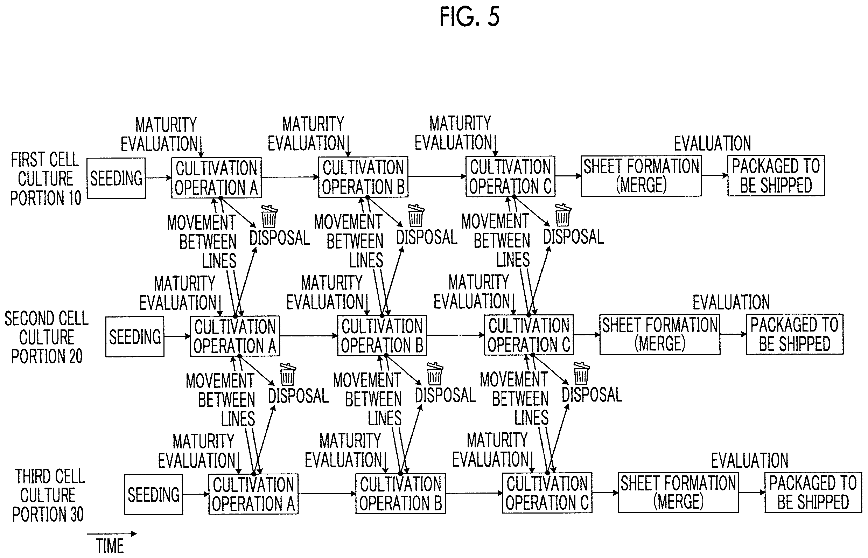

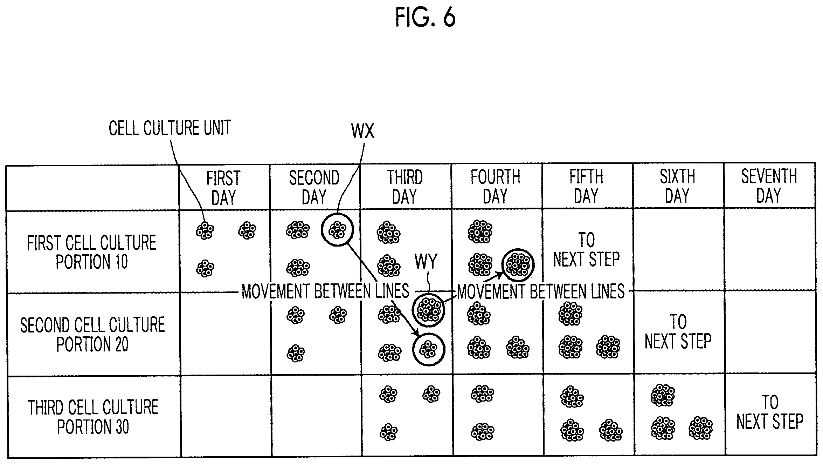

Fig. 5 is a diagram schematically illustrating a flow from the start of seeding of cell colonies in the first to third cell culture portions 10, 20, and 30 to making sheets with the cell colonies after culture and packaging the cell colonies to be shipped. In Fig. 5, time passes from the left to the right. Fig. 6 is a diagram illustrating movement of wells (cell culture units) between cell culture portions for easier understanding. In Figs. 5 and 6, the movements of the wells between the cell culture portions are indicated as movements between lines.

First, cell colonies are seeded in respective wells of the culture vessels 2 respectively installed in the first to third cell culture portions 10, 20, and 30. According to the embodiment, periods for seeding cell colonies for respective culture portions are postponed (S10). If the seeding periods are postponed, culture periods at a predetermined point of time can be postponed for each cell culture portion. That is, after cell colonies are seeded in the first cell culture portion 10, cell colonies are seeded in the second cell culture portion 20 after a constant period of time d, and cell colonies are seeded in the third cell culture portion 30 after the constant period of time d.

According to the embodiment, the first to third cell culture portions 10, 20, and 30 are provided, that is, three cell culture portions are provided. However, three or more cell culture portions may be provided. In this case, cell colonies are seeded in the respective cell culture portions while the seeding is postponed by the constant period of time d. The periods of time from the start of seeding to the end of the culture of the respective cell culture portions are the same.

Also, in each of the first to third cell culture portions 10, 20, and 30, maturity of cell colonies of the respective wells of the culture vessel 2 from the start of seeding for each predetermined period of time is evaluated. The evaluation interval of maturity from the start of seeding may be the same interval or may be different intervals in the first to third cell culture portions 10, 20, and 30. As illustrated in Fig. 5, the times for evaluating maturity are desirably evaluated according to the time for performing any cultivation operations A to C on the culture vessel 2 before or after the operations are performed. Examples of the cultivation operation include exchange or addition of culture medium, but the other operations may also be performed. Fig. 6 illustrates an example of performing evaluation of maturity for each day respectively in the first to third cell culture portions 10, 20, and 30. Hereinafter, a method of evaluating maturity of cell colonies of respective wells is specifically described.

First, the culture vessel 2 installed in the cell culture portion is transported to the image pick-up portion 40 by the transport portion 12 and images of respective wells of the culture vessel 2 are picked up (S12). The magnification of the optical system 41 at this point is set to low magnification, for example, about four times.

Also, image signals of respective wells of which images are picked up in low magnification by the image pick-up portion 40 are input to the maturity evaluation portion 50, and the maturity evaluation portion 50 obtains respective evaluation values of the maturity based on the image signals of the respective wells (S14). Specifically, according to the embodiment, sizes of the cell colonies included in the image signals of the respective wells are obtained as evaluation values of the maturity.

The evaluation values of the respective wells obtained by the maturity evaluation portion 50 are output to the movement target cell determining portion 60, and the movement target cell determining portion 60 first determines whether the evaluation values of the respective wells are included in a first threshold value range between a first upper upper threshold value and a first lower threshold value illustrated in Fig. 3 (S16). In a case where evaluation values are in the first threshold value range (S16, YES), the cell colonies in the wells having the evaluation values are normally mature and thus culture is continued in the present cell culture portion without change.

Meanwhile, in a case where evaluation values are not in the first threshold value range (S16, NO), the movement target cell determining portion 60 further determines whether the evaluation values are included in a second threshold value range between the second upper threshold value and the second lower threshold value illustrated in Fig. 3 (S18). In a case where the evaluation values are not in the second threshold value range, the movement target cell determining portion 60 evaluates that the cell colonies in the wells having the evaluation values do not normally mature and determines the wells as the targets to be moved (S24). When the evaluation values are compared with the first threshold value range or the second threshold value range, in a case where the evaluation values extremely deviate from the threshold value range, cell colonies in the well having the evaluation values may be disposed. The range of the evaluation values for determining disposal targets may be predetermined. The disposal of the cell colonies are performed by the cell moving portion 13 as described above.

Meanwhile, in S18, in a case where the evaluation values are in the second threshold value range (S18, YES), images of the wells having the evaluation values are picked up at high magnification (S20). The magnification at this point is, for example, set as about 20 times. In a case where the images are picked up at high magnification, images of the entire range of the wells cannot be picked up with image pick-up of one time. Therefore, the range of the wells is divided into a plurality of image pick-up ranges and images are picked up for respective image pick-up ranges. According to the embodiment, since the wells to be high magnification image pick-up targets can be narrowed down by performing determination using threshold value ranges in two stages, image pick-up time can be reduced.

The image signals obtained by picking up images of the wells at high magnification are output to the maturity evaluation portion 50, and the maturity evaluation portion 50 calculates evaluation values again, based on the input image signals. In a case where evaluation values are first calculated based on low magnification images, sizes of the cell colonies are used as evaluation values, that is evaluation values are obtained in cell colony units. However, when evaluation values are calculated based on the high magnification images, evaluation values are obtained in respective cell units. Specifically, according to the embodiment, for example, the number of cells and the cell density are obtained as evaluation values.

The evaluation values of the wells obtained again by the maturity evaluation portion 50 are output to the movement target cell determining portion 60 and the movement target cell determining portion 60 determines whether the evaluation values are included in the third threshold value range (not illustrated) (S22). The third threshold value range is also predetermined depending on types of evaluation values.

In a case where the evaluation values are in the third threshold value range (S22, YES), the movement target cell determining portion 60 evaluates that the cell colonies having the evaluation values in the wells are normally mature and causes culture in the present cell culture portion to be continued without change.

Meanwhile, also, in a case where the evaluation values are not included in the third threshold value range (S22, NO), the movement target cell determining portion 60 evaluates that cell colonies having the evaluation values in the wells are not normally mature and determines wells as the targets to be moved (S24). When the evaluation values and the third threshold value range compared with each other, in a case where evaluation values are greater than these threshold value ranges, the cell colonies having the evaluation values in the wells may be disposed. The range of the evaluation values determined as the disposal targets may be predetermined.

As described above, with respect to the respective first to third cell culture portions 10, 20, and 30, evaluation values of maturity of the respective wells in the culture vessel 2 are obtained, and it is determined whether the respective wells are targets to be moved based on the evaluation values.

The wells determined as the targets to be moved are moved to the other cell culture portions depending on sizes of the evaluation values. Specifically, as illustrated in Fig. 6, in a case where, in the evaluation of the first cell culture portion 10 on the second day of which seeding time is earliest, evaluation values of a predetermined well WX are smaller than the second threshold value range or smaller than the third threshold value range, that is, in a case where maturity of the well WX is lower than the predetermined condition, the well WX is moved to the second cell culture portion 20 or the third cell culture portion 30. That is, the well WX is moved to the second cell culture portion 20 or the third cell culture portion 30 of which a remaining culture period is longer than the first cell culture portion 10. Accordingly, the cell colonies of the well WX can be cultured to sufficient sizes. Whether the well WX is to be moved to the second cell culture portion 20 or to be moved to the third cell culture portion 30 is determined based on the sizes of the evaluation values.

For example, in a case where maturity of the well WX in the first cell culture portion 10 is greater than the second threshold value range or greater than the third threshold value range, that is, in a case where the maturity of the well WX is higher than the predetermined condition, the well WX is disposed since a cell culture portion for adjusting the maturity does not exist.

For example, as illustrated in Fig. 6, in evaluation of the second cell culture portion 20 on the third day of which a seeding time is second earliest, in a case where evaluation values of a predetermined well WY are greater than the second threshold value range or greater than the third threshold value range, that is, in a case where the maturity of the well WY is higher than the predetermined condition, the well WY is moved to the first cell culture portion 10. That is, the well WY is moved to the first cell culture portion 10 of which the remaining culture period is shorter than the second cell culture portion 20. Accordingly, the growth of the cell colonies of the the well WY can be suppressed.

Meanwhile, in the evaluation of the second cell culture portion 20 on the third day, in a case where the evaluation values of the predetermined well WY are smaller than the second threshold value range or smaller than the third threshold value range, that is, in a case where the maturity of the well WY is lower than the predetermined condition, the well WY is moved to the third cell culture portion 30. That is, the well WY is moved to the third cell culture portion 30 of which the remaining culture period is longer than the second cell culture portion 20. Accordingly, the cell colonies of the well WY can be cultured to sufficient sizes.

With respect to the evaluation of the predetermined time of the third cell culture portion 20 of which the seeding time is slowest, in a case where evaluation values of a predetermined well WZ (not illustrated) are greater than the second threshold value range or greater than the third threshold value range, that is, in a case where maturity of the well WZ is higher than the predetermined condition, the well WZ is moved to the second cell culture portion 20. That is, the well WZ is moved to the second cell culture portion 20 of which a remaining culture period is shorter than the third cell culture portion 30. Accordingly, the growth of the cell colonies of the the well WY can be suppressed.

In a case where the maturity of the well WZ in the third cell culture portion 30 is smaller than the second threshold value range or smaller than the third threshold value range, that is, in a case where maturity of the well WZ is lower than the predetermined condition, the well WZ is disposed since a cell culture portion for adjusting the maturity does not exist.

If wells are moved between the cell culture portions based on the sizes of the evaluation values of the wells to be moved as described above, the maturity of the cell colonies in the respective wells in the respective culture vessels 2 installed in the respective cell culture portions 10, 20, and 30 is caused to be even.

With respect to the first to third cell culture portions 10, 20, and 30, in a case where periods of time are completed from the start of seeding to the end of the culture, the cell colonies of respective wells in the culture vessels 2 are merged, are made to sheets, and packaged to be shipped.

According to the embodiment, images are picked up at two types of low magnification and high magnification. However, the magnification is not limited to two types, images may be picked up at two or more types of magnification, determination may be performed with evaluation values and threshold value ranges of maturity depending on the magnification.

Subsequently, the cell culture device and the cell culture method according to the second embodiment are described. Basic configurations of a cell culture device 3 according to the second embodiment are the same as those of the cell culture device 1 according to the first embodiment illustrated in Fig. 1. With respect to the cell culture device 1 according to the first embodiment, maturity of the cell colonies at a predetermined point of time of the respective cell culture portions is varied by postponing the start times of seeding in the first to third cell culture portions 10, 20, and 30. In contrast, in the cell culture device 3 according to the second embodiment, maturity of cell colonies of respective cell culture portions at predetermined point of time is caused to be different by causing culture speeds of the first to third cell culture portions 10, 20, and 30 to be different speeds.

Accordingly, in the cell culture device 3 according to the second embodiment, concentrations of culture liquids (culture media) used in the respective culture vessels 2 respectively installed in the first to third cell culture portions 10, 20, and 30 are caused to be different concentrations. For example, concentrations of culture liquids of the culture vessels 2 in the first cell culture portion 10 are caused to be relatively low concentrations, and the concentrations of the culture liquids of the culture vessels 2 in the second cell culture portion 20 are caused to be relatively intermediate concentration, and concentrations of the culture liquids of the culture vessels 2 in the third cell culture portion 30 are caused to be relatively high concentrations. Accordingly, the first cell culture portion 10 performs low speed culture, the second cell culture portion 20 performs intermediate speed culture, and the third cell culture portion 30 performs high speed culture. The other configurations are the same as those of the cell culture device 1 according to the first embodiment.

In the cell culture device 3 according to the embodiment, conditions of the concentrations of the culture liquids in the respective cell culture portions are caused to be different. However, the invention is not limited to this and other culture conditions may be caused to be different. Specifically, the culture conditions may be any conditions, as long as the culture conditions are conditions that influence on the culture speeds of the cell colonies. Examples thereof include conditions such as types of culture media, exchange frequency of the culture media, types of scaffolding, scaffolding applying concentration to the culture medium, temperature, humidity, types of light sources used, illuminance of light source, shaking conditions of stages, oxygen concentration, or carbon dioxide concentration. At least one condition among the respective cell culture portions may be caused to be different.

For example, culture speeds may be controlled by appropriately selecting the types of the culture media from a basic fibroblast growth factor (bFGF), glutamine, and ascorbic acid depending on the types of cells.

With respect to the exchange frequency of the culture medium, as the exchange frequency of the culture medium increases, the culture speed becomes faster. Therefore, as the culture speed of the cell culture portion is a higher speed, the exchange frequency of the culture medium is controlled to higher.

With respect to the temperature, the culture speed is fastest around 35°C, and thus the temperature of the third cell culture portion 30 with the highest speed is set as 35°C. In a case where for example, the culture speed in 37°C is higher than the culture speed in 33°C, the temperature of the second cell culture portion 20 is set as 37°C, and the temperature of the first cell culture portion 10 may be set as 33°C.

With respect to the illuminance of the light source, as the illuminance is higher, the culture speed becomes faster. Therefore, as the culture speed of the cell culture portion is a higher speed, the illuminance is controlled to be increased. With respect to the types of the light source in the same manner, as the culture speed of the cell culture portion is a higher speed, the type of light source of which culture speed is a higher speed is used.

With respect to the oxygen concentration, as the oxygen concentration increases, the culture speed becomes faster. Therefore, as the culture speed of the cell culture portion is a higher speed, the oxygen concentration is controlled to be higher.

With respect to the carbon dioxide concentration, the culture speed becomes highest at the concentration of about 5%, the carbon dioxide concentration of the third cell culture portion 30 with highest speed is set as 5%, and concentrations of the first and second cell culture portions 10 and 20 are controlled to be peripheral concentrations with 5% as a center.

The types of scaffolding may be appropriately selected from vitronectin, laminin, and collagen depending on types of the cells, such that the culture speed is controlled.

With respect to the scaffolding applying concentration, as the concentration is higher, the culture speed becomes faster. Therefore, as the culture speed of the cell culture portion is a higher speed, the concentration may be set as to be higher.

The method of controlling culture condition is appropriately selected depending on the change of the culture speed according to the types of the cells and the change of the culture condition of the cells.

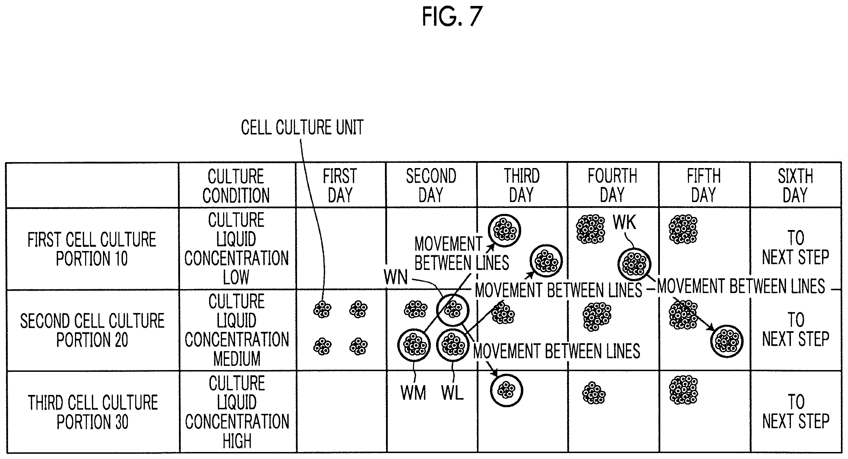

Subsequently, the movement of the wells in the cell culture device 3 according to the second embodiment is described with reference to Fig. 7. The evaluation of the maturity of the respective wells and the determination of the wells to be moved according to the second embodiment are the same as those in the first embodiment. Fig. 7 illustrates an example in which cell colonies are seeded only in the culture vessel 2 of the second cell culture portion 20 and the cell colonies are moved to the first or third cell culture portion 10 or 30 depending on the maturity. According to the second embodiment, the cell colonies do not necessarily be seeded in all the cell culture portions.

Also in the cell culture device 3 according to the second embodiment, in the same manner as the first embodiment, wells determined as targets to be moved are moved to other cell culture portions depending on the size of the evaluation values thereof. Specifically, for example, as illustrated in Fig. 7, in the evaluation of the second cell culture portion 20 on the second day of which the culture speed is intermediate speed, in a case where maturity of predetermined wells WM and WL is higher than the predetermined condition, the wells WM and WL are moved to the first cell culture portion 10. That is, the wells WM and WL are moved to the first cell culture portion 10 of which the culture speed is slower than the second cell culture portion 20. Accordingly, the growth of the cell colonies of the wells WM and WL can be suppressed.

Meanwhile, in the evaluation of the second cell culture portion 20 on the second day, in a case where maturity of a predetermined well WN is lower than the predetermined condition, the well WN is moved to the third cell culture portion 30. That is, the well WN is moved to the third cell culture portion 30 of which the culture speed is faster than the second cell culture portion 20. Accordingly, the cell colonies of the well WN can be cultured to sufficient sizes at a point of time of the end of the culture.

As illustrated in Fig. 7, in the evaluation of the first cell culture portion 10 on the fourth day of which the culture speed is low speed, in a case where the maturity of a predetermined well WK is lower than the predetermined condition, the well WK is moved to the second cell culture portion 20 or the third cell culture portion 30. That is, the well WK is moved to the second cell culture portion 20 or the third cell culture portion 30 of which the culture speed is faster than the first cell culture portion 10. Accordingly, even the cell colonies of the well WK can be cultured to sufficient sizes, at a point of time of the end of the culture. Whether the cell colonies of the well WK are to be moved to the second cell culture portion 20 or to be moved to the third cell culture portion 30 is determined based on the sizes of the evaluation values.

In the evaluation of the first cell culture portion 10 of which the culture speed is slowest at the predetermined point of time, in a case where the maturity of the wells is higher than the predetermined condition, a cell culture portion for adjusting maturity does not exist, the wells are disposed. In the evaluation of the third cell culture portion 30 of which culture speed is fastest at a predetermined point of time, even in a case where the maturity of the wells is lower than the predetermined condition, the cell culture portion for adjusting the maturity exists, and thus the wells are disposed.

As described above, according to the second embodiment, if the wells are moved between the cell culture portions based on the maturity of the wells to be moved, maturity of the respective wells in the respective culture vessels 2 installed in the respective cell culture portions 10, 20, and 30 is caused to be even.

According to the second embodiment, in a case where the period of time until the end of the culture ends, the cell colonies of respective wells in the culture vessels 2 are merged, are made to sheets, and packaged to be shipped.

According to the first embodiment, the start times of seeding of the respective cell culture portions 10, 20, and 30 are postponed, such that culture speeds of the respective cell culture portions 10, 20, and 30 are changed according to the second embodiment. However, both of the start times of seeding and the culture speeds of the respective cell culture portions 10, 20, and 30 may be changed.

According to the first and second embodiments, the culture conditions of all of the cell culture portions 10, 20, and 30 are caused to be different. However, the invention is not limited thereto, and culture conditions of at least two cell culture portions may be different from each other. Otherwise, culture conditions of all of the cell culture portions 10, 20, and 30 are caused to be the same, culture speeds of the respective cell culture portions are estimated from the maturity of the cell colonies and cell culture units may be moved.

According to the first and second embodiments, at the time of determination based on evaluation values of the maturity obtained from the low magnification images, the wells of which the evaluation values are not included in the second threshold value range are moved to other cell culture portions. However, the invention is not limited thereto, the maturity may be adjusted in the present cell culture portion without moving the wells to the other cell culture portions.

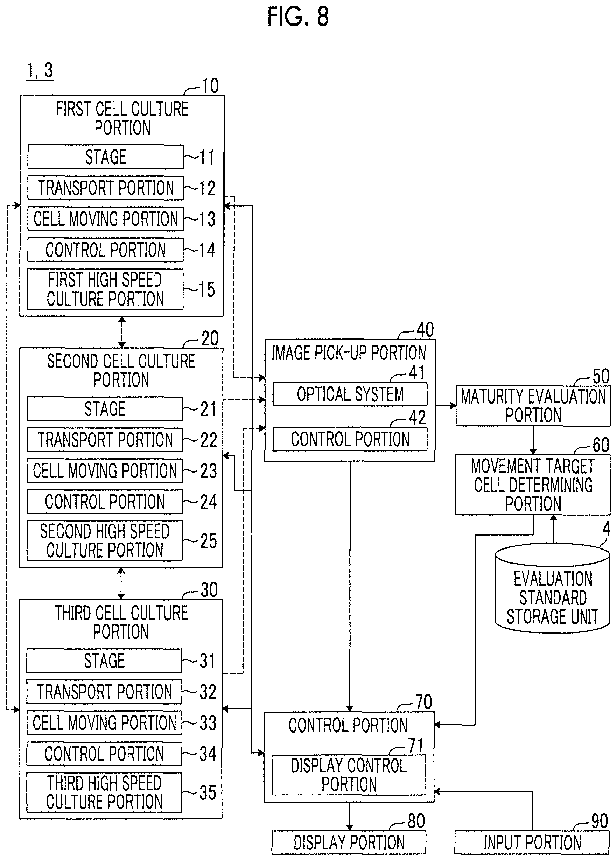

Specifically, for example, as illustrated in Fig. 8, first to third high speed culture portions 15, 25, and 35 of which the culture speed is a higher speed than the culture speed of the cells of the first to third cell culture portions 10, 20, and 30 are provided in the first to third cell culture portions 10, 20, and 30. For example, in a case where wells having evaluation values smaller than the second threshold value range exist, that is, in a case where wells of which maturity is extremely low exist, the wells are respectively moved to each of the first to third high speed culture portion 15, 25, or 35 provided in the respective cell culture portions 10, 20, and 30, the wells are cultured in each of the first to third high speed culture portion 15, 25, or 35 until evaluation values of the maturity are included in the second threshold value range or the first threshold value range. Thereafter, the wells may be returned to each of the cell culture portion 10, 20, or 30.

Configurations of the first to third high speed culture portions 15, 25, and 35 are the same as the first to third cell culture portions 10, 20, and 30, and the culture conditions described above are set such that culture proceeds at the higher speed than the first to third cell culture portions 10, 20, and 30.

In the descriptions of the first and second embodiments, respective wells of the culture vessel 2 are used as the cell culture units, but the respective cell colonies may be used as the cell culture units. That is, evaluation values of maturity are obtained with respect to the respective cell colonies, whether the cell colonies are targets to be moved is determined based on the evaluation values, the cell colonies determined as targets to be moved may be moved to other cell culture portions. With respect to high magnification image pick-up in a case where the cell colonies are used as the cell culture units, a plurality of times of image pick-up as described above may not be performed, and only one time of image pick-up may be performed.

As the method of moving the cell colonies, for example, in a case where the culture medium is solid, the cell colonies may be removed from the culture medium by a laser or the like and may be moved by sucking the cell colonies with a sucking mechanism. In a case where the culture medium is a liquid, the cell colonies may be moved by sucking the cell colonies with a sucking mechanism.

The culture vessel 2 may be the cell culture unit. In this case, in a case where a plurality of cell colonies exist in the culture vessel 2, average values, maximum values, minimum values, median values, or the like of the evaluation values of the respective cell colonies may be used in order to determine whether the culture vessel is a target to be moved.

According to the first and second embodiments, a well plate having six wells is used as a culture vessel, but the number of wells is not limited thereto. The culture vessel is not limited to the well plate, and suspension culture may be performed by using a flask, a pack, a tank, or the like. In a case where a flask, a pack, a tank, or the like is used, a flask, a pack, a tank, or the like can be a cell culture unit or respective cell colonies can be set to be cell culture units.

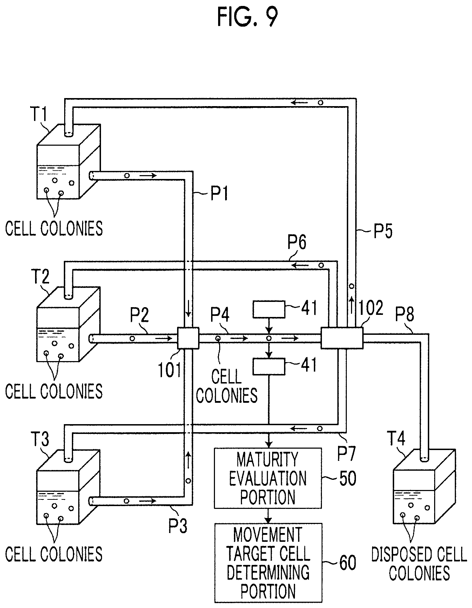

Fig. 9 is a diagram schematically illustrating a cell culture device in a case where a tank is used as a culture vessel. First to third tanks T1, T2, and T3 illustrated in Fig. 9 are stored in respective first to third cell culture portions so as to perform culture. First to third flow channels P1 to P3 for transporting cell colonies in the tank to the optical system 41 are connected to the first to third tanks T1, T2, and T3. The first to third flow channels P1 to P3 are connected to a first valve mechanism 101.

The first valve mechanism 101 connects the first flow channel P1 and a fourth flow channel P4 when maturity of cell colonies in the first tank T1 is evaluated, connects the second flow channel P2 and the fourth flow channel P4 when maturity of cell colonies in the second tank T2 is evaluated, and connects the third flow channel P3 and the fourth flow channel P4 when maturity of cell colonies in the third tank T3 is evaluated. According to the embodiment illustrated in Fig. 9, the first to fourth flow channels P1 to P4, the first valve mechanism 101, and pump mechanisms (not illustrated) provided in the first to fourth flow channels P1 to P4 correspond to the transport portions 12, 22, and 32 according to the embodiments above.

The optical system 41 according to the first and second embodiments is provided on the fourth flow channel P4 connected to the first valve mechanism 101, images of the cell colonies that flow the fourth flow channel P4 are picked up by the optical system 41, and the image signals are output to the maturity evaluation portion 50. Images of the image pick-up timing of the cell colonies may be picked up at a constant interval, or the cell colonies may be detected to be in the image pick-up range such that images are picked up. At the time of high magnification image pick-up, a plurality of times of image pick-up as in the embodiments above are not performed, and one time of image pick-up is performed.

The evaluation results in the maturity evaluation portion 50 are output to the movement target cell determining portion 60, and whether the cell colonies are targets to be moved is determined. The operations of the maturity evaluation portion 50 and the movement target cell determining portion 60 are the same as those in the first and second embodiments.

A second valve mechanism 102 is connected to the fourth flow channel P4. A fifth flow channel P5, a sixth flow channel P6, a seventh flow channel P7, and an eighth flow channel P8 are connected to the second valve mechanism 102. The first tank T1 is connected to an end of the fifth flow channel P5, the second tank T2 is connected to an end of the sixth flow channel P6, the third tank T3 is connected to an end of the seventh flow channel P7, and a fourth tank T4 in which cell colonies to be disposed are stored is connected to an end of the eighth flow channel P8.

In a case where the maturity of the cell colonies of the first tank T1 is evaluated, and it is not determined that the cell colonies are targets to be moved, the second valve mechanism 102 connects the fourth flow channel P4 and the fifth flow channel P5 and returns the cell colonies to the first tank T1. In the same manner, in a case where the maturity of the cell colonies of the second tank T2 is evaluated and it is not determined that the cell colonies are targets to be moved, the second valve mechanism 102 connects the fourth flow channel P4 and the sixth flow channel P6 and returns the cell colonies to the second tank T2. In a case where maturity of the cell colonies of the third tank T3 are evaluated and it is not determined that the cell colonies are targets to be moved, the second valve mechanism 102 connects the fourth flow channel P4 and the seventh flow channel P7 and returns the cell colonies to the third tank T3. As a result of the evaluation of the cell colonies, in a case where it is determined that the cell colonies are disposal target, the second valve mechanism 102 connects the fourth flow channel P4 and the eighth flow channel P8.

Meanwhile, for example, in a case where maturity of the cell colonies of the first tank T1 is evaluated and it is determined that the cell colonies are targets to be moved, the second valve mechanism 102 connects the fourth flow channel P4 to the sixth flow channel P6 or the seventh flow channel P7 according to the sizes of the evaluation values, and the cell colonies of the first tank T1 are moved to the second tank T2 or the third tank T3. In the same manner, in a case where maturity of the cell colonies of the second tank T2 is evaluated and it is determined that the cell colonies are the target to be moved, the second valve mechanism 102 connects the fourth flow channel P4 to the fifth flow channel P5 or the seventh flow channel P7 according to the sizes of the evaluation values, and the cell colonies of the second tank T2 are moved to the first tank T1 or the third tank T3. In a case where the maturity of the cell colonies of the third tank T3 is evaluated and it is determined the cell colonies are targets to be moved, the second valve mechanism 102 connects the fourth flow channel P4 to the fifth flow channel P5 or the sixth flow channel P6 according to the sizes of the evaluation values, and the cell colonies of the third tank T3 are moved to the first tank T1 or the second tank T2. According to the embodiment illustrated in Fig. 9, the second valve mechanism 102, the fifth to seventh flow channels P5 to P7, and pump mechanisms (not illustrated) provided in the fifth to seventh flow channels P5 to P7 correspond to the cell moving portion 13, 23, or 33 according to the embodiment.

As described above, even in a case of the suspension culture, cell colonies can be moved to other tanks having different culture conditions depending on the evaluation values of the maturity of the cell colonies in the respective tanks, and the growth of the cell colonies in the respective tanks can be suppressed. The embodiment of the suspension culture also includes the control portion 70, the display portion 80, and the input portion 90 according to the first and second embodiments, but the control portion 70, the display portion 80, and the input portion 90 are not illustrated in Fig. 9.

### Explanation of References

1, 3: cell culture device
2: culture vessel
4: evaluation standard storage unit
10: first cell culture portion
20: second cell culture portion
30: third cell culture portion
12, 22, 32: transport portion
13, 23, 33: cell moving portion
14: control portion
40: image pick-up portion
41: optical system
42: control portion
50: maturity evaluation portion
60: movement target cell determining portion
70: control portion
71: display control portion
80: display portion
90: input portion
101: valve mechanism
102: valve mechanism

## Claims

1. A cell culture device comprising:
a plurality of cell culture portions operable to respectively culture a plurality of cell culture units;
a maturity evaluation portion operable to respectively evaluate maturity of the respective cell culture units present in the respective cell culture portions during operation of the cell culture device;
a movement target cell determining portion operable to determine a cell culture unit that does not satisfy a predetermined condition as a target to be moved to another cell culture portion in a case where the cell culture unit of which maturity does not satisfy the predetermined condition among a plurality of cell culture units cultured in any one cell culture portion of the plurality of cell culture portions exists; and
a cell moving portion operable to move the cell culture unit determined as the target to be moved to the other cell culture portion,
wherein the movement target cell determining portion is operable to determine the cell culture unit as the target to be moved to another cell culture portion based on the sizes of the evaluation values, and
wherein the movement target cell determining portion is operable to cause the maturity of the respective cell culture units to be even by determining the respective cell culture units cultured in the respective cell culture portions among a plurality of cell culture portions as the target to be moved.

2. The cell culture device according to claim 1, which is operable to set
culture conditions of at least two cell culture portions to be different from each other.

3. The cell culture device according to claim 1 or 2, which is operable to set
start times of culturing at least two cell culture portions to be different from each other.

4. The cell culture device according to any one of claims 1 to 3, which is operable to set
culture conditions that influence on culture speeds of at least two cell culture portions to be different from each other.

5. The cell culture device according to claim 4,
wherein the conditions that influence on culture speeds include at least one of culture medium concentrations, types of culture media, exchange frequencies of culture media, types of scaffolding, scaffolding applying concentrations to culture media, temperature, humidity, types of light sources, illuminance of light sources, shaking condition, oxygen concentration, or carbon dioxide concentrations.

6. The cell culture device according to any one of claims 1 to 5,
wherein the maturity evaluation portion is operable to evaluate maturity of the respective cell culture units based on at least one of the number of cells, cell density, shapes of cells, sizes of cells, movement speeds of cells, proliferation speeds of cells, the number of cell colonies, sizes of cell colonies, information on shapes of cell colonies, which are obtained during operation of the cell culture device based on images of the respective cell culture units, or information on components discharged from cells obtained by interpreting culture medium components of the respective cell culture units.

7. The cell culture device according to any one of claims 1 to 6,
wherein the maturity evaluation portion is operable to evaluate maturity based on images obtained by capturing cell culture units at two or more types of magnification.

8. The cell culture device according to any one of claims 1 to 7,
wherein the movement target cell determining portion is operable to determine whether maturity of the respective cell culture units satisfies the predetermined condition by using a standard deviation or a variance of maturity of all of the cell culture units cultured in any one cell culture portion during operation of the cell culture device.

9. The cell culture device according to any one of claims 1 to 7, further comprising:
an evaluation standard storage unit operable to store relationships between the culture periods of the cell culture units and evaluation standards of maturity corresponding to the culture periods,
wherein the movement target cell determining portion is operable to obtain the evaluation standard as the predetermined condition based on a present culture period and the relationships.

10. The cell culture device according to any one of claims 1 to 9, which is operable to set
start times of culturing at least two cell culture portions to be different from each other, and
in a case where a cell culture unit of which maturity is lower than the predetermined condition exists during operation of the cell culture device, the movement target cell determining portion is operable to determine the cell culture unit that does not satisfy the condition as a target to be moved to a cell culture portion of which the start timing of the culture is later than the any one cell culture portion.

11. The cell culture device according to any one of claims 1 to 9, which is operable to set
culture conditions that influence on culture speeds of at least two cell culture portions to be different from each other,
in a case where a cell culture unit of which maturity is lower than the predetermined condition exists during operation of the cell culture device, the movement target cell determining portion is operable to determine that a cell culture unit that does not satisfy the condition is a target to be moved to a cell culture portion having a culture condition of which a culture speed is faster than that of the any one cell culture portion.

12. The cell culture device according to claim 11,
wherein in a case where a cell culture unit that does not satisfy both of the predetermined condition and a condition different from the predetermined condition exists during operation of the cell culture device, the cell moving portion is operable to remove the cell culture unit that does not satisfy the both conditions from the cell culture portion.

13. The cell culture device according to any one of claims 1 to 12, further comprising:
a notification portion operable to notify information of the cell culture unit determined as a target to be moved.

14. The cell culture device according to any one of claims 1 to 13,
wherein the cell culture unit present during operation of the cell culture device is a cell colony unit, a well unit in a culture vessel, or a culture vessel unit.

15. The cell culture device according to any one of claims 1 to 14, further comprising:
a high speed culture portion operable to culture the cell culture unit of which maturity does not satisfy the predetermined condition until the maturity reaches the condition and that is a cell culture portion adapted to have a culture speed which is a higher speed than those of the plurality of cell culture portions.

16. A cell culture method, comprising:
respectively culturing a plurality of cell culture units in a plurality of cell culture portions;
respectively evaluating maturity of the respective cell culture units in the respective cell culture portions;
automatically determining a cell culture unit that does not satisfy a predetermined condition as a cell culture unit to be moved to another cell culture portion in a case where the cell culture unit of which maturity does not satisfy the predetermined condition among a plurality of cell culture units cultured in any one cell culture portion of the plurality of cell culture portions exists; and
moving the cell culture unit determined as the target to be moved to the other cell culture portion,
wherein in a step of determining the cell culture unit, the cell culture unit is determined as the target to be moved to another cell culture portion based on the sizes of the evaluation values, and
wherein in a step of determining the cell culture unit, the maturity of the respective cell culture units is caused to be even by determining the respective cell culture units cultured in the respective cell culture portions among a plurality of cell culture portions as the target to be moved.

17. The cell culture method according to claim 16, further comprising:
culturing the cell culture unit of which maturity does not satisfy the predetermined condition until the maturity reaches the condition in a high speed culture portion which is a cell culture portion of which a culture speed is a higher speed than those of the plurality of cell culture portions.

## Patentansprüche

1. Zellkulturvorrichtung aufweisend:
eine Mehrzahl von Zellkulturbereichen, die jeweils dafür ausgelegt sind, eine Mehrzahl von Zellkultureinheiten zu kultivieren;
einen Reifebeurteilungsbereich, der dafür ausgelegt ist, jeweils die Reife der jeweiligen Zellkultureinheiten, die sich während des Betriebs der Zellkulturvorrichtung in den jeweiligen Zellkulturbereichen befinden, zu beurteilen;
einen Zielzellenverlagerungs-Bestimmungsbereich, der dafür ausgelegt ist, eine Zellkultureinheit, die eine vorbestimmte Bedingung nicht erfüllt, als ein in einen anderen Zellkulturbereich zu verlagerndes Ziel zu bestimmen in einem Fall, in dem es die Zellkultureinheit, deren Reife die vorbestimmte Bedingung nicht erfüllt, unter einer Mehrzahl von Zellkultureinheiten, die in irgendeinem Zellkulturbereich der Mehrzahl von Zellkulturbereichen kultiviert werden, gibt; und
einen Zellenverlagerungsbereich, der dafür ausgelegt ist, die Zellkultureinheit zu verlagern, die als das in den anderen Zellkulturbereich zu verlagernde Ziel bestimmt wurde,
wobei der Zielzellenverlagerungs-Bestimmungsbereich dafür ausgelegt ist, die Zellkultureinheit auf der Basis der Größen der Beurteilungswerte als das in einen anderen Zellkulturbereich zu verlagernde Ziel zu bestimmen, und
wobei der Zielzellenverlagerung-Bestimmungsbereich dafür ausgelegt ist, durch Bestimmung der jeweiligen Zellkultureinheiten, die in den jeweiligen Zellkulturbereichen unter einer Mehrzahl von Zellkulturbereichen kultiviert werden, als das zu verlagernde Ziel zu bewirken, dass die Reife der jeweiligen Zellkultureinheiten einheitlich ist.

2. Zellkulturvorrichtung nach Anspruch 1,
die dafür ausgelegt ist, zu veranlassen, dass die Kulturbedingungen von mindestens zwei Zellkulturbereichen voneinander verschieden sind.

3. Zellkulturvorrichtung nach Anspruch 1 oder 2,
die dafür ausgelegt ist, zu veranlassen, dass die Startzeiten des Kultivierens von mindestens zwei Zellkulturbereichen voneinander verschieden sind.

4. Zellkulturvorrichtung nach einem der Ansprüche 1 bis 3,
die dafür ausgelegt ist, zu veranlassen, dass Kulturbedingungen, die Kulturgeschwindigkeiten beeinflussen, von mindestens zwei Zellkulturbereichen voneinander verschieden sind.

5. Zellkulturvorrichtung nach Anspruch 4,
wobei die Bedingungen, die Kulturgeschwindigkeiten beeinflussen, mindestens eine der Bedingungen Kulturmedium-Konzentrationen, Arten von Kulturmedien, Austauschhäufigkeiten von Kulturmedien, Gerüstarten, Gerüstanwendungskonzentrationen auf Kulturmedien, Temperatur, Feuchtigkeit, Arten von Lichtquellen, Beleuchtungsstärken von Lichtquellen, Rüttelbedingung, Sauerstoffkonzentration oder Kohlendioxidkonzentrationen umfassen.

6. Zellkulturvorrichtung nach einem der Ansprüche 1 bis 5,
wobei der Reifebeurteilungsbereich dafür ausgelegt ist, die Reife der jeweiligen Zellkultureinheiten auf der Basis mindestens eines der Kriterien Anzahl an Zellen, Zelldichte, Formen der Zellen, Größen der Zellen, Bewegungsgeschwindigkeiten der Zellen, Proliferationsgeschwindigkeiten der Zellen, der Anzahl an Zellkolonien, Größen der Zellkolonien, Information bezüglich Formen von Zellkolonien, die während des Betriebs der Zellkulturvorrichtung auf der Basis von Bildern der jeweiligen Zellkultureinheiten erhalten werden, oder Information bezüglich von Zellen ausgeschiedenen Komponenten, die durch Interpretation von Kulturmedienkomponenten der jeweiligen Zellkultureinheiten erhalten wird, zu beurteilen.

7. Zellkulturvorrichtung nach einem der Ansprüche 1 bis 6,
wobei der Reifebeurteilungsbereich dafür ausgelegt ist, die Reife auf der Basis von Bildern, die durch Erfassen von Zellkultureinheiten bei zwei oder mehr Vergrößerungsarten erhalten wurden, zu beurteilen.

8. Zellkulturvorrichtung nach einem der Ansprüche 1 bis 7,
wobei der Zielzellenverlagerung-Bestimmungsbereich dafür ausgelegt ist, durch Verwendung einer Standardabweichung oder einer Varianz der Reife aller Zellkultureinheiten, die in irgendeinem Zellkulturbereich während des Betriebs der Zellkulturvorrichtung kultiviert werden, zu bestimmen, ob die Reife der jeweiligen Zellkultureinheiten die vorbestimmte Bedingung erfüllt.

9. Zellkulturvorrichtung nach einem der Ansprüche 1 bis 7, außerdem aufweisend:
eine Beurteilungsstandard-Speichereinheit, die dafür ausgelegt ist, Beziehungen zwischen den Kulturzeitspannen der Zellkultureinheiten und Reifebeurteilungsstandards, die den Kulturzeitspannen entsprechen, zu speichern,
wobei der Zielzellenverlagerung-Bestimmungsbereich dafür ausgelegt, den Beurteilungsstandard als die vorbestimmte Bedingung auf der Basis der vorliegenden Kulturzeitspanne und den Beziehungen zu erhalten.

10. Zellkulturvorrichtung nach einem der Ansprüche 1 bis 9, die dafür ausgelegt ist, zu veranlassen, dass
die Startzeiten des Kultivierens von mindestens zwei Zellkulturbereichen voneinander verschieden sind, und
in einem Fall, in dem es während des Betriebs der Zellkulturvorrichtung eine Zellkultureinheit gibt, deren Reife geringer ist als die vorbestimmte Bedingung, der Zielzellenverlagerung-Bestimmungsbereich dahingehend wirkt, die Zellkultureinheit, die die Bedingung nicht erfüllt, als das in einen Zellkulturbereich, dessen Startzeit des Kultivierens später ist als in dem einen Zellkulturbereich, zu verlagernde Ziel zu bestimmen.

11. Zellkulturvorrichtung nach einem der Ansprüche 1 bis 9, die dafür ausgelegt ist, zu veranlassen, dass
Kulturbedingungen, die Kulturgeschwindigkeiten beeinflussen, von mindestens zwei Zellkulturbereichen voneinander verschieden sind,
in einem Fall, in dem es während des Betriebs der Zellkulturvorrichtung eine Zellkultureinheit gibt, deren Reife geringer ist als die vorbestimmte Bedingung, der Zielzellenverlagerung-Bestimmungsbereich dahingehend wirkt, zu bestimmen, dass eine Zellkultureinheit, die die Bedingung nicht erfüllt, ein in einen Zellkulturbereich mit einer Kulturbedingung, deren Kulturgeschwindigkeit schneller ist als die des einen Zellkulturbereichs, zu verlegendes Ziel ist.

12. Zellkulturvorrichtung nach Anspruch 11,
bei der in einem Fall, in dem es während des Betriebs der Zellkulturvorrichtung eine Zellkultureinheit gibt, die sowohl die vorbestimmte Bedingung als auch eine von der vorbestimmten Bedingung verschiedene Bedingung nicht erfüllt, der Zellenverlagerungsbereich dahingehend wirkt, die Zellkultureinheit, die beide Bedingungen nicht erfüllt, aus dem Zellkulturbereich zu entfernen.

13. Zellkulturvorrichtung nach einem der Ansprüche 1 bis 12, außerdem aufweisend:
einen Anzeigebereich, der dafür ausgelegt ist, Information zu der Zellkultureinheit, die als das zu verlagernde Ziel bestimmt wurde, anzuzeigen.

14. Zellkulturvorrichtung nach einem der Ansprüche 1 bis 13,
bei der die Zellkultureinheit, die während des Betriebs der Zellkulturvorrichtung vorliegt, eine Zellkolonieeinheit, eine Vertiefungseinheit in einem Kulturgefäß oder eine Kulturgefäßeinheit ist.

15. Zellkulturvorrichtung nach einem der Ansprüche 1 bis 14, außerdem aufweisend:
einen Hochgeschwindigkeitskulturbereich, der dafür ausgelegt ist, die Zellkultureinheit, deren Reife die vorbestimmte Bedingung nicht erfüllt, zu kultivieren bis die Reife die Bedingung erreicht, und der ein Zellkulturbereich ist, der daran angepasst ist, eine Kulturgeschwindigkeit zu haben, die eine höhere Geschwindigkeit als jene der Mehrzahl von Zellkulturbereichen ist.

16. Zellkulturverfahren aufweisend:
jeweils Kultivieren einer Mehrzahl von Zellkultureinheiten in einer Mehrzahl von Zellkulturbereichen;
jeweils Beurteilen der Reife der jeweiligen Zellkultureinheiten in den jeweiligen Zellkulturbereichen;
automatisch Bestimmen einer Zellkultureinheit, die eine vorbestimmte Bedingung nicht erfüllt, als eine in einen anderen Zellkulturbereich zu verlagernde Zellkultureinheit in einem Fall, in dem es unter einer Mehrzahl von Zellkultureinheiten, die in irgendeinem Zellkulturbereich der Mehrzahl von Zellkulturbereichen kultiviert werden, eine Zellkultureinheit gibt, deren Reife die vorbestimmte Bedingung nicht erfüllt; und
Verlagern der Zellkultureinheit, die als das zu verlagernde Ziel bestimmt wurde, in den anderen Zellkulturbereich,
wobei in einem Schritt des Bestimmens der Zellkultureinheit die Zellkultureinheit auf der Basis der Größen der Beurteilungswerte als das in einen anderen Zellkulturbereich zu verlagernde Ziel bestimmt wird, und
wobei in einem Schritt des Bestimmens der Zellkultureinheit durch Bestimmen der jeweiligen Zellkultureinheiten, die in den jeweiligen Zellkulturberichen aus einer Mehrzahl von Zellkulturbereichen kultiviert werden, als das zu verlagernde Ziel veranlasst wird, dass die Reife der jeweiligen Zellkultureinheiten einheitlich ist.

17. Zellkulturverfahren nach Anspruch 16, außerdem aufweisend:
Kultivieren der Zellkultureinheit, deren Reife die vorbestimmte Bedingung nicht erfüllt, in einem Hochgeschwindigkeitskulturbereich, der ein Zellkulturbereich ist, dessen Kulturgeschwindigkeit eine höhere Geschwindigkeit ist als diejenige der Mehrzahl der Zellkulturbereiche, bis die Reife die Bedingung erreicht.

## Revendications

1. Dispositif de culture cellulaire, comprenant :
une pluralité de portions de culture cellulaire pouvant fonctionner pour cultiver respectivement une pluralité d'unités de culture cellulaire ;
une portion d'évaluation de maturité pouvant fonctionner pour évaluer respectivement la maturité des unités de culture cellulaire respectives présentes dans les portions de culture cellulaire respectives durant le fonctionnement du dispositif de culture cellulaire ;
une portion de détermination de cellule cible pour déplacement pouvant fonctionner pour déterminer une unité de culture cellulaire, laquelle ne satisfait pas une condition prédéterminée, comme cible à déplacer vers une autre portion de culture cellulaire dans un cas où l'unité de culture cellulaire dont la maturité de satisfait pas la condition prédéterminée parmi une pluralité d'unités de culture cellulaire cultivées dans une portion de culture cellulaire quelconque parmi la pluralité de portions de culture cellulaire existe, et
une portion de déplacement de cellule pouvant fonctionner pour déplacer l'unité de culture cellulaire déterminée comme la cible à déplacer vers l'autre portion de culture cellulaire ;
dans lequel la portion de détermination de cellule cible pour déplacement peut fonctionner pour déterminer l'unité de culture cellulaire comme cible à déplacer vers une autre portion de culture cellulaire, sur la base des tailles des valeurs d'évaluation, et
dans lequel la portion de détermination de cellule cible pour déplacement peut fonctionner pour rendre la maturité des unités de culture cellulaire respectives égales en déterminant les unités de culture cellulaire respectives cultivées dans les portions de culture cellulaire respectives parmi une pluralité de portions de culture cellulaire comme cible à déplacer.

2. Dispositif de culture cellulaire selon la revendication 1, lequel peut fonctionner pour régler des conditions de culture d'au moins deux portions de culture cellulaire de manière à ce qu'elles soient différentes les unes des autres.

3. Dispositif de culture cellulaire selon la revendication 1 ou 2, lequel peut fonctionner pour régler des temps de début de culture d'au moins deux portions de culture cellulaire de manière à ce qu'ils soient différents les uns des autres.

4. Dispositif de culture cellulaire selon l'une quelconque des revendications 1 à 3, lequel peut fonctionner pour régler des conditions de culture, lesquelles ont une influence sur les vitesses de culture d'au moins deux portions de culture cellulaire de manière à ce qu'elles soient différentes les unes des autres.

5. Dispositif de culture cellulaire selon la revendication 4,
dans lequel les conditions influençant des vitesses de culture incluent au moins un élément parmi des concentrations de milieu de culture, des types de milieux de culture, des fréquences d'échange de milieux de culture, des types d'échafaudage, des concentrations d'application d'échafaudage à des milieux de culture, la température, l'humidité, les types de sources lumineuses, l'éclairement de sources lumineuses, une condition de secousses, la concentration en oxygène, ou des concentrations en dioxyde de carbone.

6. Dispositif de culture cellulaire selon l'une quelconque des revendications 1 à 5,
dans lequel la portion d'évaluation de maturité peut fonctionner pour évaluer la maturité des unités de culture cellulaire respectives sur la base d'au moins un élément parmi le nombre de cellules, la densité de cellules, les formes de cellules, les tailles de cellule, les vitesses de déplacement de cellules, les vitesses de prolifération de cellules, le nombre de colonies cellulaires, les tailles de colonies cellulaires, des informations sur des formes de colonies cellulaires, lesquelles sont obtenues durant le fonctionnement du dispositif de culture cellulaire sur la base d'images des unités de culture cellulaire respectives, ou des informations sur des composants déchargés de cellules obtenues en interprétant des composants de milieu de culture des unités de culture cellulaire respectives.

7. Dispositif de culture cellulaire selon l'une quelconque des revendications 1 à 6,
dans lequel la portion d'évaluation de maturité peut fonctionner pour évaluer la maturité sur la base d'images obtenues en capturant des unités de culture cellulaire sur deux types de grossissement ou plus.

8. Dispositif de culture cellulaire selon l'une quelconque des revendications 1 à 7,
dans lequel la portion de détermination de cellule cible pour déplacement peut fonctionner pour déterminer si la maturité des unités de culture cellulaire respectives satisfait la condition prédéterminée en utilisant un écart type ou une variance de maturité de toutes les unités de culture cellulaire dans une portion de culture cellulaire quelconque durant le fonctionnement du dispositif de culture cellulaire.

9. Dispositif de culture cellulaire selon l'une quelconque des revendications 1 à 7, comprenant en outre :
une unité de stockage de référence d'évaluation pouvant fonctionner pour stocker des relations entre les périodes de culture des unités de culture cellulaire et des références d'évaluation de maturité correspondant aux périodes de culture,
dans lequel la portion de détermination de cellule cible pour déplacement peut fonctionner pour obtenir la référence d'évaluation comme condition prédéterminée sur la base d'une période de culture présente et des relations.

10. Dispositif de culture cellulaire selon l'une quelconque des revendications 1 à 9, lequel peut fonctionner pour régler
des temps de début de culture d'au moins deux portions de culture cellulaire de manière à ce qu'ils soient différents les uns des autres, et
dans un cas où il existe une unité de culture cellulaire dont la maturité est inférieure à la condition prédéterminée durant le fonctionnement du dispositif de culture cellulaire, la portion de détermination de cellule cible pour déplacement peut fonctionner pour déterminer l'unité de culture cellulaire qui ne satisfait pas la condition comme cible à déplacer vers une portion de culture cellulaire dont la temporisation de début de la culture est plus tardive que celle de la une portion de culture cellulaire quelconque.

11. Dispositif de culture cellulaire selon l'une quelconque des revendications 1 à 9, lequel peut fonctionner pour régler
des conditions de culture, lesquelles ont une influence sur des vitesses de culture d'au moins deux portions de culture cellulaire de manière à ce qu'elles soient différentes les unes des autres, et
dans un cas où il existe une unité de culture cellulaire dont la maturité est inférieure à la condition prédéterminée durant le fonctionnement du dispositif de culture cellulaire, la portion de détermination de cellule cible pour déplacement peut fonctionner pour déterminer qu'une unité de culture cellulaire qui ne satisfait pas la condition est une cible à déplacer vers une portion de culture cellulaire présentant une condition de culture dont une vitesse de culture est plus rapide que celle de la une portion de culture cellulaire quelconque.

12. Dispositif de culture cellulaire selon la revendication 11,
dans lequel dans un cas où il existe une unité de culture cellulaire qui ne satisfait pas à la fois la condition prédéterminée et une condition différente de la condition prédéterminée durant le fonctionnement du dispositif de culture cellulaire, la portion de déplacement de cellule peut fonctionner pour éliminer l'unité de culture cellulaire qui ne satisfait pas les deux conditions de la portion de culture cellulaire.

13. Dispositif de culture cellulaire selon l'une quelconque des revendications 1 à 12, comprenant en outre :
une portion de notification pouvant fonctionner pour notifier des informations de l'unité de culture cellulaire déterminée comme cible à déplacer.

14. Dispositif de culture cellulaire selon l'une quelconque des revendications 1 à 13,
dans lequel l'unité de culture cellulaire présente durant le fonctionnement du dispositif de culture cellulaire est une unité de colonie cellulaire, une unité à puits dans un récipient de culture, ou une unité de récipient de culture.

15. Dispositif de culture cellulaire selon l'une quelconque des revendications 1 à 14, comprenant en outre :
une portion de culture à vitesse élevée pouvant fonctionner pour cultiver l'unité de culture cellulaire dont la maturité ne satisfait pas la condition prédéterminée jusqu'à ce que la maturité atteigne la condition, et laquelle est une portion de culture cellulaire apte à présenter une vitesse de culture supérieure à celles de la pluralité de portions de culture cellulaire.

16. Procédé de culture cellulaire, comprenant les étapes suivantes :
cultiver respectivement une pluralité d'unités de culture cellulaire dans une pluralité de portions de culture cellulaire ;
évaluer respectivement la maturité des unités de culture cellulaire respectives dans les portions de culture cellulaire respectives ;
déterminer automatiquement une unité de culture cellulaire, laquelle ne satisfait pas une condition prédéterminée, comme unité de culture cellulaire à déplacer vers une autre portion de culture cellulaire dans un cas où l'unité de culture cellulaire dont la maturité de satisfait pas la condition prédéterminée parmi une pluralité d'unités de culture cellulaire cultivées dans une portion de culture cellulaire quelconque parmi la pluralité de portions de culture cellulaire existe, et
déplacer l'unité de culture cellulaire déterminée comme la cible à déplacer vers l'autre portion de culture cellulaire ;
dans lequel lors d'une étape de détermination de l'unité de culture cellulaire, l'unité de culture cellulaire est déterminée comme cible à déplacer vers une autre portion de culture cellulaire, sur la base des tailles des valeurs d'évaluation, et
dans lequel lors d'une étape de détermination de l'unité de culture cellulaire, la maturité des unités de culture cellulaire respectives est rendue égale en déterminant les unités de culture cellulaire respectives cultivées dans les portions de culture cellulaire respectives parmi une pluralité de portions de culture cellulaire comme cible à déplacer.

17. Procédé de culture cellulaire selon la revendication 16, comprenant en outre l'étape pour :
cultiver l'unité de culture cellulaire dont la maturité ne satisfait pas la condition prédéterminée jusqu'à ce que la maturité atteigne la condition dans une portion de culture à vitesse élevée, laquelle est une portion de culture cellulaire dont une vitesse de culture est une vitesse supérieure à celles de la pluralité de portions de culture cellulaire.
